# EUROPEAN PATENT APPLICATION

(11) **EP 2 308 498 A1**
(43) Date of publication of application: **13.04.2011**
(21) Application number: 09171840.3
(22) Date of filing: 30.09.2009
(51) Int. Cl.: A61K 35/74, A61P 1/00, A61P 29/00

(54) **Administration of Bifidobacterium breve during infancy to prevent inflammation later in life**

(71) Applicant: Nestec S.A., 1800 Vevey (CH)
(72) Inventor: García-Rodenas, Clara Lucia, 1072, FOREL (CH); Nutten, Sophie, 1005, LAUSANNE (CH); Mercenier, Annick, 1030, BUSSIGNY (CH); Prioult, Guénolée, 1005, LAUSANNE (CH); Bergonzelli, Gabriela, 1030, BUSSIGNY (CH); Benyacoub, Jalil, 1010, LAUSANNE (CH); Philippe, David, 1000, LAUSANNE 25 (CH)
(74) Representative: Marquardt, Ulf

(57) **Abstract**

The present invention relates to the prevention of inflammatory disorders. In particular, the present invention relates to compositions that can be administered to a subject early in life and that will protect the subject from inflammatory disorders later in life. One embodiment of the present invention is a composition comprising *Bifidobacterium breve* to be administered to mothers during pregnancy and/or lactation and/or to infants for preventing inflammatory disorders of the infant occurring at least two months after the administration of this strain or even later in life.

## Description

The present invention relates to the prevention of inflammatory disorders. In particular, the present invention relates to compositions that can be administered to a subject early in life and that will protect the subject from inflammatory disorders later in life. One embodiment of the present invention is a composition comprising *Bifidobacterium breve* to be administered to mothers during pregnancy and/or lactation and/or to infants for preventing inflammatory disorders of the infant occurring at least two months after the administration of this strain or even later in life.

There is accumulating evidence supporting that events occurring early in life may have medium- and long term effects on health and disease. Several epidemiological studies suggest a role of perinatal events in the etiology of different inflammatory disorders. In particular, alterations in the early microbiota colonization of the gut due to e.g. antibiotic treatment, birth by caesarean section and/or preterm birth, by impairing the maturation of key mechanisms of the intestinal barrier and immune defenses, can contribute to the increased prevalence of inflammatory diseases observed during the last decades.

Inflammation is recognized as a type of nonspecific immune response and is a physiological way by which the body reacts to infection, irritation or other injury. Typical symptoms of inflammations are redness, warmth, swelling and pain. Usually, inflammation is tightly regulated by the body. However, if the control pathways of inflammation fail, and inflammation runs uncontrolled, it can lead to several disorders at various locations in the body, such as Inflammatory Bowel Disease (IBD), gastritis, gastroenteritis, colitis, celiac disease, eczema, psoriasis, allergy, rheumatoid arthritis, or autoimmune diseases. Mild inflammation is also involved in the pathogenesis of functional gastrointestinal disorders (FGID), such as Irritable Bowel Syndrome (IBS), functional dyspepsia, functional constipation, functional diarrhoea, functional abdominal pain, functional bloating, epigastric pain syndrome, and also in gastro-esophageal reflux, and infantile colics.

Inflammatory disorders can be treated or prevented by many means. Since inflammatory disorders may be very different from one another, the treatment methods are usually dependant on the cause of inflammation. Typically, inflammation is treated today by the administration of non-steroidal anti-inflammatory drugs such as NSAIDs, or corticosteroids. Inflammatory disorders and their medication may however have serious consequences, so that it must be preferred to avoid the occurrence of inflammatory disorders in the first place.

The administration of probiotics has been proposed as a prophylactic/therapeutic tool in the management of inflammatory diseases. For example, a highly concentrated cocktail of eight live probiotic bacteria has been shown to be effective in prevention (Gionchetti, P., et al., 2003, Gastroenterology 124:1202-1209) and treatment of recurrent or refractory pouchitis in humans (Gionchetti, P., et al., 2000, Gastroenterology 119:305-309; Mimura, T., et al., 2004, Gut 53:108-114). However, other studies have shown poor efficacy of the probiotic administration (Rolfe et al, Cochrane Database Syst. Rev. 2006 (4): CD004826). Furthermore, the protective effect of the probiotics rapidly disappears after discontinuation of the treatment (Rosenfeldt et al, J Pediatr 2004, 145(5): 612-6; Guyonnet et al, Br J Nutr, Epub ahead of print:¨; doi:10.1017/S0007114509990882).

Consequently, the administration of probiotics is today seen as a short term tool to reduce the severity of inflammation and/or to prevent relapse during the remission phases of an already established and diagnosed inflammatory disorder.

It would however be desirable to have available a composition that can limit the susceptibility acquired during early life to develop inflammatory disease and that can be used efficiently for the mid and long-term prevention of inflammatory disorders.

The present inventors have addressed this need.

It was consequently the object of the present invention to provide the art with a composition that can be used as part of a nutritional strategy to provide a mid- and long-term protection against inflammatory disorders that is safe, healthy and easy to administer and produces no unwanted side effects.

Mid-term protection means a protective effect lasting from 2 months to 1 year after administration.

Long-term protection means a protective effect lasting for more than 1 year after administration.

The present inventors were surprised to see that they could achieve this object by the subject matter of the independent claim. The dependent claims further develop the present invention.

The present inventors have found that - surprisingly and contrary to what is known about the mid- and long term protective effect of probiotics - the administration of the probiotic *Bifidobacterium breve* achieves this object.

The inventors believe that the administration of *Bifidobacterium breve, e.g.,* during the perinatal period, allows minimizing changes in the intestinal barrier maturation induced by an altered early microbiota colonization, that could be induced, e.g., by an early antibiotic therapy, or by cesarian delivery and is often found in preterm and low birth weight infants. Avoiding changes in the intestinal barrier maturation, will serve to prevent inflammatory disorders later in life.

The present invention hence concerns also nutritional strategies comprising the administration of *Bifidobacterium breve* that promote gut barrier maturation, in particular in situations where the intestinal colonization by microbiota is impaired and, consequently are able to durably prevent an increased susceptibility to inflammation later in life.

The present invention relates hence to the use of the probiotic *Bifidobacterium breve,* for example during infancy, to produce long-lasting health benefits.

Notably, this preventive effect against inflammatory disorders is maintained long after the administration of probiotics is interrupted.

Hence, the subject matter of the present invention allows producing a preventive effect against inflammatory disorders independently from the fact whether or not probiotics are presently consumed when an inflammatory challenge is being encountered.

The composition of the present invention will show an effect even if it was administered a long time before the preventive effect is needed.

One embodiment of the present invention relates to a composition comprising *Bifidobacterium breve* to be administered to mothers during pregnancy for use in the prevention of inflammatory disorders of the infant occurring at least two months later in life.

The present invention also relates to a composition comprising *Bifidobacterium breve* to be administered to mothers during lactation for use in the prevention of inflammatory disorders of the infant occurring at least two months later in life.The protective effect will be maintained even after the end of lactation.

Further, the present invention relates to a composition comprising *Bifidobacterium breve* to be administered to infants for use in the prevention of inflammatory disorders of the infant occurring at least two months later in life. The composition will be in particular effective if it is administered to a 0-2 year old infant.

"Later in life" means at least 2 months after the end of the administration of the composition of the present invention, for example at least 6 months after the end of the administration of the composition of the present invention, further for example at least 2 years after the end of the administration of the composition of the present invention, even further for example at least 5 years after the end of the administration of the composition of the present invention, for example at least 10 years after the end of the administration of the composition of the present invention, such as at least 40 years after the end of the administration of the composition of the present invention.

The composition of the present invention may be a food product, a nutritional composition, a nutraceutical, a drink, a food additive an animal food product or a pharmaceutical composition.

For example, the composition may be a human milk fortifier, an infant feeding formula, a growing up milk, an infant cereal, a baby food, a food supplement, a liquid suspension, a powder, a tablet, a tooth paste, a gum or candy.

A food additive or a pharmaceutical composition may be in the form of tablets, capsules, pastilles or a liquid for example.

The compositions may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents, gel forming agents, antioxidants and antimicrobials. They may also contain conventional pharmaceutical additives and adjuvants, excipients and diluents, including, but not limited to, water, gelatine of any origin, vegetable gums, ligninsulfonate, talc, sugars, starch, arabic gum, vegetable oils, polyalkylene glycols, flavouring agents, preservatives, stabilizers, emulsifying agents, buffers, lubricants, colorants, wetting agents, fillers, and the like.

The composition of the present invention may contain in addition vitamins, minerals, trace elements and other micronutrients in accordance with the recommendations of Government bodies such as the USRDA.

For example, the composition may contain per daily dose one or more of the following micronutrients in the ranges given:- 300 to 500 mg calcium, 50 to 100 mg magnesium, 150 to 250 mg phosphorus, 5 to 20 mg iron, 1 to 7 mg zinc, 0.1 to 0.3 mg copper, 50 to 200 µg iodine, 5 to 15 µg selenium, 1000 to 3000 µg beta carotene, 10 to 80 mg Vitamin C, 1 to 2 mg Vitamin B1, 0.5 to 1.5 mg Vitamin B6, 0.5 to 2 mg Vitamin B2, 5 to 18 mg niacin, 0.5 to 2.0 µg Vitamin B12, 100 to 800 µg folic acid, 30 to 70 µg biotin, 1 to 5 µg Vitamin D, 3 to 10 µg Vitamin E.

A stabilizing agent may be added to stabilize the composition and its constituents.

A flavouring agent and/or a colouring agent may be added to adjust flavours and to give the composition a colour that is easy to identify and/or that is perceived as pleasant.

Prebiotics may be added.

"Prebiotic" means non-digestible food substances that promote the growth of health beneficial micro-organisms and/or probiotics in the intestines. They are not broken down in the stomach and/or upper intestine or absorbed in the GI tract of the person ingesting them, but they are fermented by the gastrointestinal microbiota and/or by probiotics. Prebiotics are for example defined by Glenn R. Gibson and Marcel B. Roberfroid, Dietary Modulation of the Human Colonic Microbiota: Introducing the Concept of Prebiotics, J. Nutr. 1995 125: 1401-1412.

Prebiotics may support the growth of *Bifidobacterium breve* and/or, in case of ingestion, may stimulate the growth of beneficial micro-organisms in the intestines. Prebiotics may also act synergistically with the viable probiotic bacteria that are present in the composition and/or that may be added.

The prebiotics that may be used in accordance with the present invention are not particularly limited and include all food substances that promote the growth of probiotics and/or health beneficial bacteria in the intestines. Preferably, they may be selected from the group consisting of oligosaccharides from milk or other sources, optionally containing sialic acid, fructose, fucose, galactose, mannose; dietary fibers, in particular soluble fibers, soy fibers; inulin; fibersol or mixtures thereof. Preferred prebiotics are sialo-oligosaccharides, fructo-oligosaccharides (FOS), galacto-oligosaccharides (GOS), isomalto-oligosaccharides (IMO), xylo-oligosaccharides (XOS), arabino-xylo oligosaccharides (AXOS), mannan oligosaccharides (MOS), oligosaccharides of soy, glycosylsucrose (GS), lactosucrose (LS), sialyl-lactose (SL) Fucosyl-lactose (FL), Lacto-N-Neotetraose (LNNT), lactulose (LA), palatinose-oligosaccharides (PAO), maltooligosaccharides, gums and/or hydrolysates thereof, pectins, starches, and/or hydrolysates thereof.

Other probiotic micro-organisms may be used in combination with the *Bifidobacterium breve* to be used in the present invention.

Preferably, such an added probiotic may be selected from the group consisting of the genera *Bifidobacterium, Lactobacillus, Lactococcus, Enterococcus, Streptococcus, Propionibacterium, Pediococcus, Escherichia coli, Debaryomyces, Kluyveromyces, Saccharoymces, Schizosaccharomyces, Zygosaccharomyces, Yarrowia, Candida,* in particular selected from the group consisting of the species *Bifidobacterium longum, Bifidobacterium lactis, Bifidobacterium animalis, Bifidobacterium breve, Bifidobacterium infantis, Bifidobacterium bifidum, Bifidobacterium adolescentis, Lactobacillus acidophilus, Lactobacillus helveticus, Lactobacillus casei, Lactobacillus paracasei, Lactobacillus salivarius, Lactobacillus plantarum, Lactobacillus fermentum, Lactobacillus johnsonii, Lactobacillus reuteri, Lactobacillus gasseri, Lactobacillus rhamnosus, Lactococcus ssp. such as Lactococcus lactis, Lactococcus crernoris, Lactococcus diacetylactis, Enterococcus faecium, Enterococcus faecalis, Saccharomyces cerevisiae, Saccharomyces boulardii, Schizosaccharomyces pombe, Kluyveromyces lactis, Yarrowia* lypolitica or mixtures thereof, preferably selected from the group consisting of *Lactobacillus johnsonii* (NCC533; CNCM I-1225), *Bifidobacterium longum* (NCC490; CNCM 1-2170), *Bifidobacterium longum* (NCC2705; CNCM 1-2618), *Bifidobacterium longum* (NCC3001; ATCC BAA-999), *Bifidobacterium lactis* (NCC2818; CNCM 1-3446), *Bifidobacterium breve* (strain A), *Lactobacillus paracasei* (NCC2461; CNCM 1-2116), *Lactobacillus rhamnosus* GG (ATCC53103), *Lactobacillus rhamnosus* LPR (NCC4007; CGMCC 1.3724), *Lactobacillus reuteri* (ATCC 55730), *Lactobacillus reuteri* (DSM 17938), *Enterococcus faecium* SF 68 (NCIMB10415), *Saccharomices boulardii,* and mixtures thereof. All these probiotics may be added in a viable or in a non-replicating form.

It is in particular preferred if the probiotics that may be added to the composition of the present invention are probiotics obtainable from human milk, such as *Lactobacillus helveticus* and *Bifidobacterium longum.*

The composition of the present invention may be intended for any mammal, but is preferably intended for humans or pets.

The composition of the present invention may contain at least one protein source, at least one carbohydrate source and/or at least one lipid source.

Any suitable dietary protein may be used, for example animal proteins (such as milk proteins, meat proteins and egg proteins); vegetable proteins (such as soy proteins, wheat proteins, rice proteins, and pea proteins); partial or total hydrolysates of these proteins, mixtures of free amino acids; or combinations thereof. If hydrolysed proteins are required, the hydrolysis process may be carried out as desired and as is known in the art. Milk proteins such as casein and whey, and soy proteins are particularly preferred. As far as whey proteins are concerned, the protein source may be based on acid whey or sweet whey or mixtures thereof and may include alpha- lactalbumin and beta-lactoglobulin in whatever proportions are desired. Preferably however, in particular if the composition is an infant feeding formula, the protein source is based on modified sweet whey.

If the composition of the present invention contains a protein source, then the amount of protein or protein equivalent in the composition is typically in the range of 1.6-7.5 g/100kcal of the composition. In particular for nutritional formulas, the protein source should provide that the minimum requirements for essential amino acid content are met.

If the composition contains a carbohydrate source, the kind of carbohydrate to be used is not particularly limited. Any suitable carbohydrate may be used, for example sucrose, lactose, glucose, fructose, corn syrup solids, maltodextrins, starch and mixtures thereof. Combinations of different carbohydrate sources may be used. The carbohydrates may preferably provide 30% to 80% of the energy of the composition. For example, the composition may comprise a carbohydrate source in an amount of 9-18 g/100kcal of the composition.

If the composition contains a lipid source, the kind of lipid to be used is not particularly limited. If the composition includes a lipid source, the lipid source may provide 5% to 70% of the energy of the composition. Long chain n-3 and/or n-6 polyunsaturated fatty acids, such as DHA, ARA and/or EPA may be added. A suitable fat profile may be obtained using a blend of canola oil, corn oil, high-oleic acid sunflower oil and medium chain triglyceride oil. The composition may comprise a lipid source in an amount of 1.5-7 g/100kcal of the composition.

The composition may be a powdered nutritional formula, for example to be reconstituted with water or milk. Such a powdered nutritional formula may have a water activity smaller than 0.2, for example in the range of 0.19-0.05, preferably smaller than 0.15. Water activity or a_{w} is a measurement of the energy status of the water in a system. It is defined as the vapor pressure of water divided by that of pure water at the same temperature; therefore, pure distilled water has a water activity of exactly one. This low water activity will allow a high survival rate of *Bifidobacterium breve* if the probiotic is used alive.

The inflammatory disorder that may be prevented by the subject matter of the present invention may be selected from systemic inflammatory disorders, gut inflammatory disorders and functional gastrointestinal disorders, gastroesophageal reflux, infantile colic; and combinations thereof.

The systemic inflammatory disorder may be selected from the group consisting of eczema, psoriasis, allergy and/or rheumatoid arthritis.

The gut inflammatory disorder may be selected from the group consisting of necrotizing enterocolitis, gastritis, gastroenteritis, colitis, Inflammatory Bowel Disease (IBD) and/or celiac disease.

The functional gastrointestinal disorders may be selected from the group consisting of infantile colic, irritable bowel syndrome; functional dyspepsia; functional constipation, functional diarrhoea; functional abdominal pain; functional bloating, Epigastric Pain Syndrome, Postprandial Distress Syndrome, gastro-esophageal reflux or combinations thereof.

The *Bifidobacterium breve strain* used in the present invention may advantageously be a *Bifidobacterium breve* obtainable from human breast milk. The *Bifidobacterium breve* may also be obtained from human breast milk.

For example, the *Bifidobacterium breve* may be *Bifidobacterium breve* NCC2950.

*Bifidobacterium breve* NCC2950 was deposited under the Budapest treaty as *Bifidobacterium breve* CNCM 1-3865.

The *Bifidobacterium breve* may be administered as viable bacterium, as non-replicating bacterium, as components of *Bifidobacterium breve* cells and/or as medium fermented with *Bifidobacterium breve.*

Using viable *Bifidobacterium breve* has the advantage that they are able to persist in the intestine and, hence, are capable of establishing their presence in the intestinal tract and multiplying their effect.

Non-replicating *Bifidobacterium breve* or components of *Bifidobacterium breve* cells or medium fermented with *Bifidobacterium breve* have the advantage that they are far easier to handle than their live counterparts. Additionally, they are far more storage stable and need less stringent packaging conditions. Non-replicating *Bifidobacterium breve* or components of *Bifidobacterium breve* cells or medium fermented with *Bifidobacterium breve* would allow developing a large variety of functional foods which by their nature do not allow the addition of live probiotics without additional measures to protect them. This plays a role for example in the provision of cereal bars, fruit juices, UHT-drinks, shelf stable drinks, etc. Further, for example in immuno-compromised customers, the use of live probiotics might be limited due to a potential risk to develop bacteremia. Additionally, the provision of non-replicating probiotic micro-organisms or components of *Bifidobacterium breve* cells or medium fermented with *Bifidobacterium breve* allows the hot reconstitution, e.g., of powdered nutritional compositions.

The composition of the present invention may be administered to the pregnant mother, the lactating mother and/or to the infant. The infant may be a term born infant or a preterm born infant.

The composition of the present invention is thought to be in particular beneficial for preterm born infants and/or low birth weight infants, as well as to infants born by cesarian section and/or to infants treated with antibiotics, as they are more likely to develop inflammatory disorders later in life

Further, advantageously, the composition may be administered to infants during the suckling and/or weaning periods and/or up to 18 months thereafter.

The composition of the present invention is also useful if administered during or after an antibiotic treatment. "After an antibiotic treatment" may relate to the time period starting within one month from the end of the antibiotic treatment.

Particular good results are achieved if the composition of the present invention is administered every day for at least 1 week, for example for at least 2 weeks, or for at least 3 weeks, or for at least 4 weeks.

In prophylactic applications, compositions according to the invention are administered to a patient susceptible to or otherwise at risk of inflammatory disorders in an amount that is sufficient to at least partially reduce the risk of developing such disorders. Such an amount is defined to be "a prophylactic effective dose". The precise amounts to be used can be determined by those skilled in the art and depend on a number of patient specific factors such as the patient's state of health, age, weight, and the specific bacterial strain used.

In general the composition of the present invention contains *Bifidobacterium breve* in a prophylactic effective dose.

The composition of the present invention may contain the *Bifidobacterium breve* in a viable and/or non-replicating form and/or components of *Bifidobacterium* breve cells and/or medium fermented with *Bifidobacterium breve.*

The composition of the present invention may contain *Bifidobacterium breve* in the range of 10² to 10¹² cfu/100kcal, preferably 10⁶ to 10¹¹ cfu/serving.

Obviously, non-replicating *Bifidobacterium breve* or components of *Bifidobacterium breve* cells or medium fermented with *Bifidobacterium breve* do not form colonies; consequently this term is to be understood as the amount of non replicating bacteria or components of *Bifidobacterium breve* cells or medium fermented with *Bifidobacterium breve* that is obtained from 10² to 10¹² cfu/g replicating *Bifidobacterium breve.* This includes *Bifidobacterium breve* that are inactivated or dead. In other words, the quantity of *Bifidobacterium breve* which the composition contains is expressed in terms of the colony forming ability of that quantity of *Bifidobacterium breve* as if all the *Bifidobacterium breve* were alive irrespective of whether they are, in fact, alive, non replicating, such as inactivated or dead, or components of *Bifidobacterium breve* cells or medium fermented with *Bifidobacterium breve* or a mixture of any or all of these states.

Consequently, if non-replicating *Bifidobacterium breve* or components of *Bifidobacterium breve* cells or medium fermented with *Bifidobacterium breve are used,* the prophylactic effective dose may be in the range of about 0,005 mg - 1000 mg non-replicating *Bifidobacterium breve* or components of *Bifidobacterium breve* cells or medium fermented with *Bifidobacterium breve* per daily dose .

The composition of the present invention may be administered in 1 - 5 servings per day.

The composition of the present invention may further be used to treat or prevent disorders linked to an impaired gut barrier maturation.

An impaired gut barrier maturation may translate into an impaired development of the luminal, epithelial and/or immune mechanisms in the gut, which - if healthy - is capable of preventing pathological antigen and microorganism translocation from the lumen to the body.

The composition in accordance with the present invention may further be used for reversing a weak early immune imprinting in the gut. A weak early immune imprinting may lead to a long-term alteration of the innate and/or acquired immune mechanisms. This may in turn lead to pathological immune responses to antigens and/or to microorganisms later in life.

Those skilled in the art will understand that they can freely combine all features of the present invention described herein, without departing from the scope of the invention as disclosed.

Further advantages and features of the present invention are apparent from the following Examples and Figures.
Figure 1 shows the design of the experiments referred to in Example 1.
Figure 2 shows the body weight loss in the groups after TNBS administration. *B. breve = B. breve* NCC2950, *B. longum = B. longum NCC* 435. Means±SEM are represented. *: significantly different (p<0.05)
Figure 3 shows the intestinal permeability to 51Cr-EDTA in the groups after TNBS administration. *B. breve* = *B. breve* NCC2950, *B. longum* = *B. longum* NCC 435. Means±SEM are represented. *: significantly different (p<0.05)
Figure 4 shows the myeloperoxidase activity in colonic mucosa in the groups after TNBS administration. *B. breve = B. breve* NCC2950, *B. longum = B. longum* NCC 435. Means±SEM are represented. *: significantly different (p<0.05)
Figure 5 shows the Wallace inflammatory macroscopic score in the colon of the groups after TNBS administration. *B. breve = B. breve* NCC2950, *B. longum* = *B. longum* NCC 435. Means±SEM are represented. *: significantly different (p<0.05)
Figure 6 shows the length of the colon in the groups after TNBS administration. *B. breve = B. breve* NCC2950, *B. longum = B. longum* NCC 435. Means±SEM are represented. *: significantly different (p<0.05)

### Example :

To demonstrate the long term protective effect of *B. breve,* e.g.,B. *breve* NCC 2950, administration during infancy, a rat model of neonatal antibiotherapy and inflammation challenge two months after the end of the probiotic treatment (i.e. this corresponds to the adult period in the rat) was used.

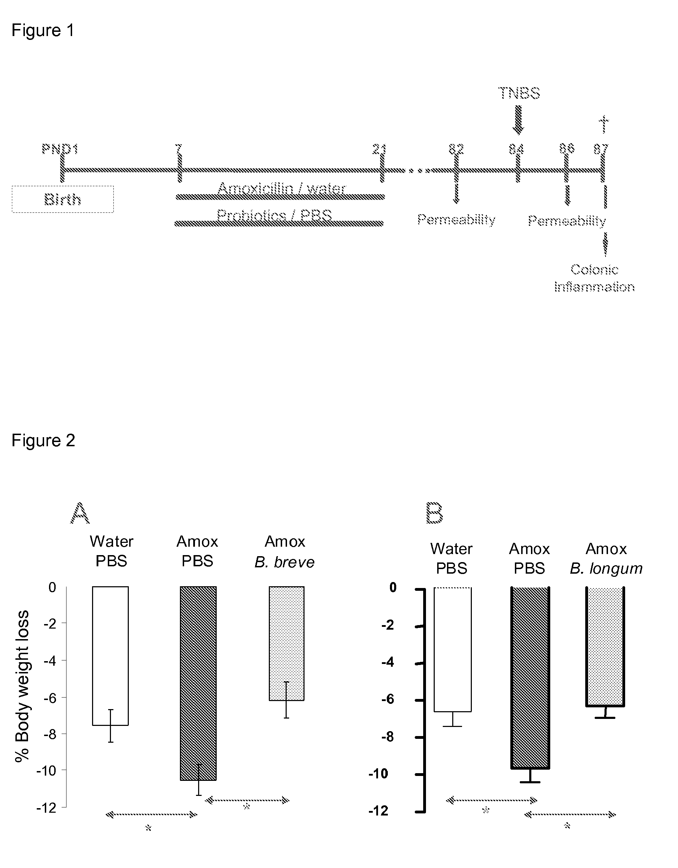

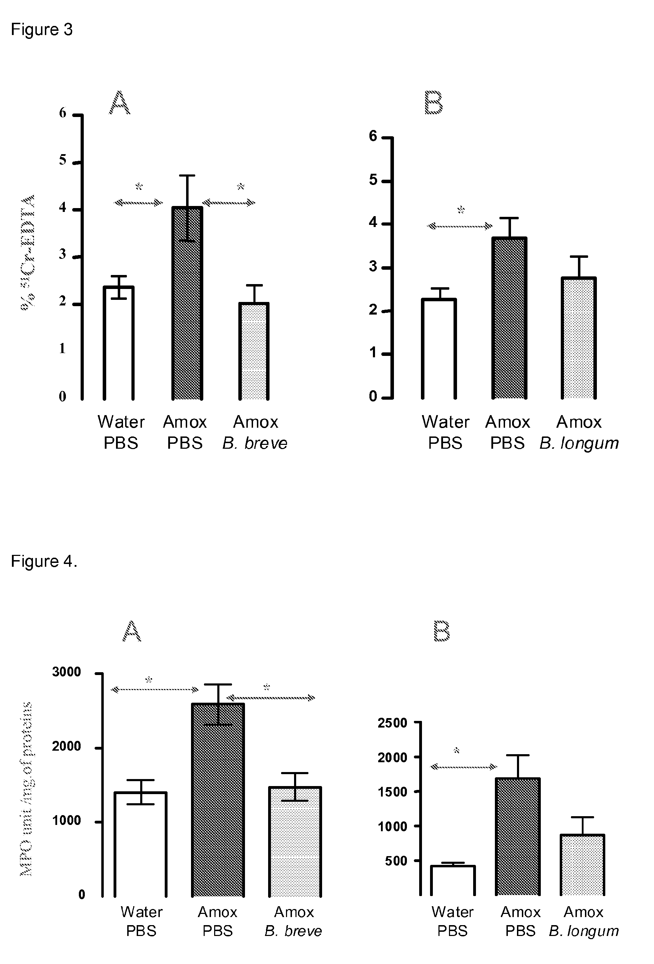

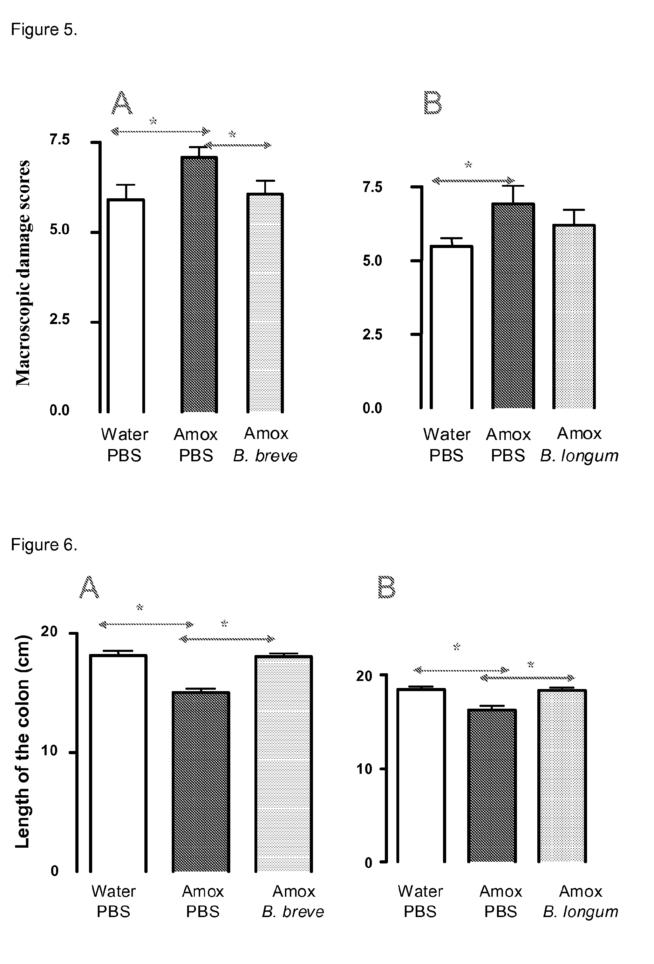

Rat pups were treated with two daily gavages of amoxicillin (Amox) or water (Water) for 15 consecutive days (from postnatal day 7 (PND7) to PND21). Two different probiotic bifidobacteria were tested in two separate experiments. In experiment A, Amox animals received a daily gavage, with either PBS (Amox-PBS group) or *B. breve* NCC 2950 (Amox-B. breve group) from PND7 to PND21. The experiment B was similar to experiment A but *B. longum* NCC 435 (Amox-B. longum group) instead of *B. breve* was provided. *B. longum* NCC 435 was deposited under the Budapest treaty as *B. longum* ATCC 15707. Sixty-mg/Kg body weight trinitrobenzosulfonic acid (TNBS) was administered intra-rectally in the colon of these animals when they reached the age of 12 weeks. Body weight was monitored daily after TNBS treatment. The integrity of the intestinal barrier was assessed by measuring *in vivo* permeability to ⁵¹Cr-EDTA and bacterial translocation to mesenteric lymph nodes (MLN), spleen and liver. Level of colonic inflammation was assessed by myeloperoxidase activity in the colonic mucosa, Wallace's inflammatory macroscopic score and length of the colon (Figure 1). *B. breve* NCC 2950 completely normalized all the studied parameters of intestinal barrier and inflammatory response (Table 1, Fig. 2A-6A). On the contrary, a proven anti-inflammatory Bifidobacterium strain such as *B. longum* NCC 435 was only partially efficient (Fig. 2B and 6B) and was not able to normalize the parameters related to the intestinal barrier (Table 1, Fig. 3B).

Table 1. Bacterial translocation to liver, spleen and MLN (Means±SEM)

| | Total aerobic bacteria (Log10 CFU/g ) | | |
|---|---|---|---|
| groups | Liver | spleen, | MLN |
| Water-PBS | - | - | - |
| Amox-PBS | 2.67 ± 2.19 | 3.31 ± 2.79 | 2.44 ± 2.24 |
| Amox-*B. breve* | 0.39 ± 0.54 | - | - |
| | | | |
| groups | Liver | Spleen | MLN |
| Water-PBS | - | N A | - |
| Amox-PBS | 2.00 ± 1.00 | NA | 0.66 ± 1.15 |
| Amox-*B.longum* | 1.72 ± 1.68 | NA | |

| | | | |
|---|---|---|---|
| *B. breve* = *B. breve* NCC2950, *B. longum* = *B. longum* NCC 435 NA: non analyzed - : below the detection limit | | | |

## Claims

1. Composition comprising *Bifidobacterium breve* to be administered to mothers during pregnancy and/or lactation and/or to infants for use in the prevention of inflammatory disorders of the infant occurring at least two months later in life.

2. Composition in accordance with claim 1, wherein the inflammatory disorder is selected from systemic inflammatory disorders, gut inflammatory disorders and functional gastrointestinal disorders; and combinations thereof.

3. Composition in accordance with one of the preceding claims, wherein the systemic inflammatory disorder is selected from the group consisting of eczema, psoriasis, allergy and/or rheumatoid arthritis; wherein gut inflammatory disorder is selected from the group consisting of necrotizing enterocolitis, gastritis, gastroenteritis, colitis, Inflammatory Bowel Disease (IBD) and/or celiac disease; wherein the gut functional disorder is selected from the group consisting of functional gastro-intestinal disorders such as functional dyspepsia, functional constipation, functional diarrhoea, functional abdominal pain, functional bloating, epigastric pain syndrome,infantile colics, gastroesopahgeal reflux, and/or combinations thereof.

4. Composition in accordance with one of the preceding claims, wherein the *Bifidobacterium breve* is obtainable from human breast milk.

5. Composition in accordance with one of the preceding claims, wherein the *Bifidobacterium breve* is *Bifidobacterium breve* NCC2950.

6. Composition in accordance with one of the preceding claims to be administered during and/or after an antibiotic treatment.

7. Composition in accordance with one of the preceding claims for use in the treatment or prevention of disorders linked to an impaired gut barrier maturation.

8. Composition in accordance with one of the preceding claims for use in reversing a weak early immune imprinting in the gut.

9. Composition in accordance with one of the preceding claims wherein said composition is a human milk fortifier, an infant feeding formula, an enteral nutrition product, a growing up milk, an infant cereal, a baby food, a food supplement, a liquid suspension, a powder, a tablet, a tooth paste, gummies or candies.

10. Composition in accordance with one of the preceding claims wherein said composition is provided to infants during the suckling and/or weaning periods and/or up to 18 months thereafter.

11. Composition in accordance with one of the preceding claims wherein said composition is provided to pregnant and/or lactating women.

12. Composition in accordance with one of the preceding claims wherein the composition contains *Bifidobacterium breve* in the range of 10² to 10¹² cfu/100kcal, preferably 10⁶ to 10¹¹ cfu/serving.

13. Composition in accordance with one of the preceding claims wherein the composition contains *Bifidobacterium breve* in a viable form.

14. Composition in accordance with one of the preceding claims wherein the composition contains *Bifidobacterium breve* in a non-replicating form and/or components of *Bifidobacterium breve* cells and/or medium fermented with *Bifidobacterium breve.*
